Europäisches Patentamt

(19) European Patent Office

Office européen des brevets

(11) Publication number: **0 267 347 B1**

# EUROPEAN PATENT SPECIFICATION

(45) Date of publication of patent specification: 21.08.91　(51) Int. Cl.⁵: **A61F 2/76**

(21) Application number: **86850394.7**

(22) Date of filing: **14.11.86**

(54) **A method and a means for adjusting and securing an artificial leg.**

(43) Date of publication of application:
**18.05.88 Bulletin 88/20**

(45) Publication of the grant of the patent:
**21.08.91 Bulletin 91/34**

(84) Designated Contracting States:
**AT BE CH DE ES FR GB GR IT LI LU NL SE**

(56) References cited:
**EP-A- 0 187 516**
**FR-A- 1 274 616**
**GB-A- 2 082 074**
**US-A- 4 221 007**
**US-A- 4 608 054**

(73) Proprietor: **Landstingens Inköpscentral LIC
ekonomisk förening
Svetsarvägen 20
S-171 83 Solna(SE)**

(72) Inventor: **Hofer, Björn
Lädersättravägen 20
S-175 70 Järfälla(SE)**

(74) Representative: **Barnieske, Hans Wolfgang
c/o H.W. Barnieske Patentbyrä AB S:ta Ragn-
hildsgatan 24-26 P.O. Box 25
S-151 21 Södertälje 1(SE)**

## Description

The present invention relates to a method and a means for adjusting and securing an artificial leg or the like. The invention also relates to a combination of an artificial leg and an adjustment means therefore, in accordance with the preamble of claim 4

In known methods and devices for securing artificial legs, the coupling arrangements are of a more or less complicated nature. Examples of such arrangements which permit a certain amount of final adjustment are shown in SE-B-446 373 (US 4 608 054). However, this known arrangement requires basic alignment of the artificial leg when the cast is taken and a "patient-jig" must therefore be used. The basic alignment can then be transferred to a "socket-jig" and is maintained during manufacture of both a plaster positive and a prosthesis socket. The basic alignment obtained when the cast is taken can then be finally adjusted when the finished prosthesis is fitted.

Another method is known from document GB-A-2 082 074. According to this method an alignment device for an artificial limb has means for attaching the device to a limb socket and an outer limb structure so as to provide a temporary, external connection between the socket and the outer limb structure. The device has joints with screw clamps to allow the relative alignment of the outer limb structure and the socket to be adjusted both rotationally and linearly, and to allow a walking trial to be performed. When a required alignment has been established, the device is used to maintain the relative positions of the socket and the outer limb structure whilst a permanent connection is formed between them, where upon the device is detached from the limb.

One drawback with these known methods is that the alignment of the artificial limb requires advanced equipment in the form of various jigs and advanced manufacturing methods for the socket. This entails high costs in manufacturing artificial limbs.

Document EP-A-0187516 discloses a device comprising three cooperating coupling members which are held together by a centre bolt. The coupling members have opposed spherically curved contact surfaces provided with rows of engaging teeth and indentations respectively, extending across each surface. The rows of engaging teeth and indentations in the two contact surfaces are arranged at right angles to each other. This known arrangement permits a certain angular adjustment before the bolt is finally tightened to keep the coupling members together. The drawback with this known arrangement is that it permits only stepwise angular adjustment and that it is not sufficiently strong.

The object of the present invention is to eliminate the drawbacks mentioned above and effect a simplified method of adjusting artificial limbs, as well as a means enabling such simplified adjustment at low cost.

This is achieved by the method defined in claim 1. Embodiments of the method are defined in claims 2 and 3.

The method according to the invention can suitably be performed using the combination of an artificial leg and of an adjusting means for the artificial leg, defined in claim 4. Embodiments of the combinations are defined in claims 5 - 9 and below.

The adjustment members suitably comprise four symmetrically located setting screws, each passing through a tapped hole in one of the parts, a spring being provided around at least two of the setting screws to provide flexible spacing between the parts.

The means may include a sliding element arranged in a transverse guide on the second part in order to support the second coupling member and permit displacement thereof. The transverse guide is preferably formed by a bushing arranged around two setting screws located opposite each other and by two adjacent pins which limit lateral movement of the sliding element.

The sliding element may also be provided with a rectangular opening into which the second coupling member shall be inserted, the length of the opening being such as to permit lateral movement of the second coupling member.

The invention will be described in more detail in the following, with reference to the accompanying drawings in which

| Figure 1 | shows a view from above of a means according to the invention, |
| Figure 2 | shows, partially in section, a view along the line II - II in Figure 1, and |
| Figure 3 | shows a view from above of a lower part of the means according to Figure 1. |

The figures show an adjustment instrument 1 with an upper ring 2 and a lower ring 3. The upper ring 2 is provided with four diametrically running tapped holes 4 for securing screws 5, and four axially running holes 6, which are provided with a counter sink for accomodating setting screws 7. The lower ring is provided with four axially running holes 8, tapped for the setting screws 7, and four holes 9 for the insertion of pins 10. The setting screws 7 are provided with a spring 11 and two opposite setting screws 7 are provided with a sliding bushing 12 arranged on the lower ring 3. The sliding bushing 12 and pins 10 form a transverse guide for a sliding element 13. The sliding element 13 is provided with a rectangular opening 14.

The adjustment instrument 1 is intended to be arranged around an artificial limb 15 including a prosthetic socket 16, a lower spacer 17 and a connection element 18. The connection element 18 consists of a number of coupling members such as a socket adapter 19, a lens 20 and a shank adapter 21. The shank adapter 21 can be secured to the lower spacer 17 by means of a clamp 22.

When performing the method according to the invention, a prosthesis socket 16 fitting the patient's leg is first manufactured. The socket 16 is provided with a socket adapter 19 which is secured to the socket by glueing, embedding in plastic, screwing or the like. Prefabricated sockets with an integrated socket adapter are also feasible. The shank adapter 21 is placed in the rectangular opening 14 of the sliding element 13. The shank adapter 21 is preferably quadratic and has an upper protruding flange, thus permitting lateral displacement along the opening 14. The lens 20 is then placed between the shank adapter 21 and socket adapter 19. The adjustment instrument 1 is then secured to the socket adapter 19 by tightening the screws 5 in the upper ring 2, thus entering recesses in the socket adapter 19. The coupling members (19,20,21) are arranged in a neutral position in relation to the adjustment instrument and the adjustment instrument 1 is finally screwed together with the aid of setting screws 7 against resistance offered by the springs 11.

The lens 20 has one flat surface in contact with the shank adapter 21 and one curved surface in contact with the socket adapter 19 with corresponding contact surfaces permitting continuous movement therebetween and thus allowing angular adjustment and lateral displacement in relation to the prosthetic socket 16.

The coupling members 19, 20 and 21 are prevented from moving in any direction by tightening of the screws 7. The lower spacer 17 together with an artificial foot is then fitted to the connection element 18. The spacer 17 may suitably consist of a pipe cut to the desired length and secured to the shank adapter 21 by the clamping means 22. In the embodiment shown this is a hose clip.

In order to adjust the angle of the artificial leg 15 one setting screw 7 must be loosened while the opposite screw 7 is tightened. The coupling members of the connection element 18 will thus be displaced along the curved contact surface until the correct angular position is achieved.

To set the correct horizontal position for the artificial limb 15, two adjacent setting screws 7 must be loosened. The shank adapter 21 can then be displaced in the plane along the rectangular opening 14 in one direction and with the aid of the sliding element 13 in the other direction. The sliding element 13 slides on the lower ring 3 and is guided by the bushings 12, whereupon movement is limited by the pins 10 and by the setting screws 7. When the correct horisontal position has been achieved, the two setting screws 7 can be tightened.

When setting the horizontal position, the coupling members of the connection element 18 are displaced along the flat contact surface. The artificial limb 15 has now been positioned correctly and the coupling members of the connection element 18 are substantially fixed in relation to each other. The patient can thus perform walking assessment and subsequent adjustment is possible if required. The coupling members of the connection element are then joined together and the adjustment instrument 1 can then be removed. The coupling members may be joined by means of a quick-drying glue, by fusion, with the aid of various types of locking pins passed through the connection element, by means of external clamps or other similar methods.

A considerable advantage of the present invention is that the adjustment instrument can be used immediately to adjust and secure other artificial limbs.

Another advantage over known technique is that the lower spacer need not be removed from the artificial limb once adjustment has been performed, and there is thus no risk of the position of the foot in relation to the leg being disturbed.

The artificial leg 15 can then be machined to remove superfluous material and the connection element can be embedded in plastic.

The present invention enables angular adjustment of about +/- 8° and lateral adjustment of about +/- 15 mm. Of course, even greater adjustment is possible using other dimensions.

The invention can naturally be modified to give further adjustment possibilites. Additional coupling members may be used, having inclined contact surfaces so that displacement of these parts would permit adjustment in vertical direction. This could also be achieved by providing the shank adapter with internal threading and a similarly tapped sleeve which is then secured to the spacer.

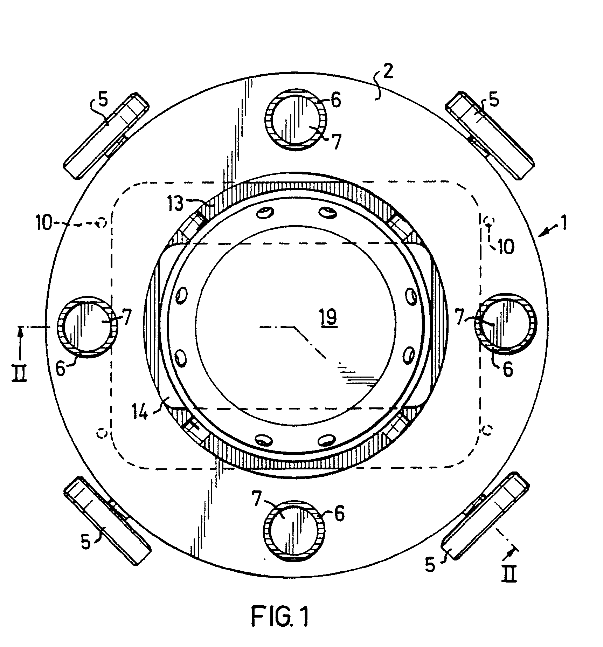

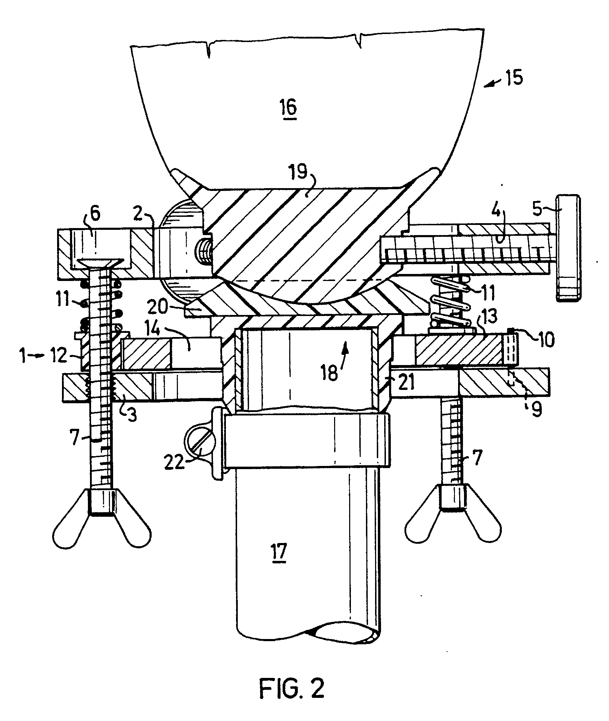

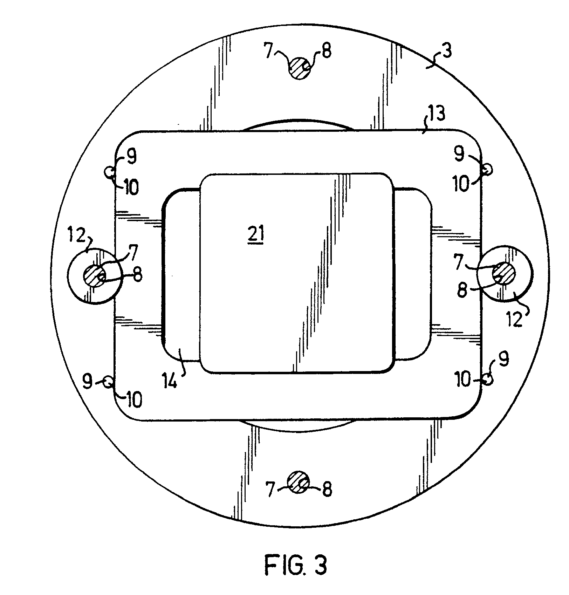

## Claims

1. A method of adjusting and securing an artificial leg (15), said artificial leg consisting of a prosthetic socket (16) arranged around the leg, a lower spacer (17) designed for connection to an artificial foot, and a connection element (18) located between the socket (16) and the lower spacer (17), **wherein** the connection element (18) consists of coupling members (19, 20, 21) displaceable in relation to each other, the outer coupling members (19, 21) of the connection element (18) being anchored to the socket (16) and the lower spacer (17), respectively, wherein an adjustment instrument (1) is applied around the connection element (18) and substantially prevents movement of the coupling members (19, 20, 21) in any direction when in the tightened state, but permits and causes relative adjusting movement thereof in its loosened state, the adjustment instrument acting directly on the coupling members (19, 20, 21) which are thereby displaced so that the artificial leg (15) is adjusted substantially correctly, and the adjustment instrument (1) is thereafter tightened in order to secure the connection element (18) wherein the coupling members (19, 20, 21) are then joined together, after which the adjustment instrument (1) can be removed.

2. A method according to claim 1, wherein at least one contact surface between the coupling mebmers (19, 20, 21) is flat in order to permit lateral movement.

3. A method according to claims 1 - 2, wherein at least one contact surface between the coupling members (19, 20, 21) is domed to permit angular movement.

4. A combination of an artificial leg and of an adjusting means for the artificial leg, said artificial leg (15) consisting of a prosthetic socket (16) arranged around the leg, a lower spacer (17) designed for connection to an artificial foot, and a connection element (18) located between the socket (16) and the lower spacer (17) the connecting element (18) consists of coupling members (19, 20, 21) displaceable in relation to each other, the outer coupling members (19, 21) of the connection element (18) being anchored to the socket (16) and the lower spacer (17), respectively, characterized in that said adjusting means comprises a first part (2) provided with securing members (5) designed to be secured to a first coupling member (19) of the connection element (18), a second part (3) designed to displaceably support a second coupling member (21) of the connection element (18) and a number of adjustment members (7) cooperating with the first and second parts (2, 3) to permit adjustment of the parts.

5. A means according to claim 4, wherein the adjustment members (7) comprise four symmetrically located setting screws, each passing through a tapped hole (8) in one of the parts (2, 3).

6. A means according to claim 5, wherein a spring (11) is provided around at least two of the setting screws (7) to provide flexible spacing between the parts (2, 3).

7. A means according to any of claims 4 - 6, wherein the means includes a sliding element (13) arranged in a transverse guide on the second part (3) in order to support the second coupling member (21) and permit displacement thereof.

8. A means according to claim 7, wherein said guide is formed by a bushing (12) arranged around two setting screws (7) arranged opposite each other and by two adjacent pins (10) which limit lateral movement of the sliding element (13).

9. A means according to claim 7 or 8, wherein the sliding element (13) is provided with a rectangular opening (14) into which the second coupling member (21) shall be inserted, the length of the opening being such as to permit lateral movement of the second coupling member (21).

**Revendications**

1. Procédé pour ajuster et fixer une jambe artificielle (15), ladite jambe artificielle comportant une douille de prothèse (16) disposée autour de la jambe, une cale d'espacement inférieure (17) conçue pour être reliée à un pied artificiel, et un élément de liaison (18) disposé entre la douille (16) et la cale d'espacement inférieure (17), dans lequel l'élément de liaison (18) comprend des éléments d'accouplement (19,20,21) mobiles l'un par rapport à l'autre, les éléments extérieurs d'accouplement (19, 21) de l'élément de liaison (18) étant amarrés à la douille (16) et à la cale d'écartement inférieure (17) respectivement, dans lequel un appareil d'ajustement (1) est appliqué autour de l'élément de liaison (18) et empêche sensiblement le mouvement des éléments d'accouplement (19,20,21) dans une direction quelconque, lorsqu'il est dans la position serrée, mais permet et provoque un mouvement relatif d'ajustement entre eux lorsqu'il est dans son état relâché, l'appareil d'ajustement agissant directement sur les éléments d'accouplement (19,20,21) qui sont ainsi déplacés de façon à ajuster la jambe artificielle (15) de manière sensiblement correcte, et l'appareil d'ajustement (1) est ensuite serré afin de maintenir l'élément de liaison (18) dans lequel les éléments d'accouplement (19,20,21) sont ensuite assemblés entre eux, après quoi l'appareil d'ajustement (1) peut être retiré.

2. Procédé selon la revendication 1, dans lequel au moins une surface de contact entre les éléments d'accouplement (19,20,21) est plate de façon à autoriser un mouvement latéral.

3. Procédé selon les revendications 1 et 2, dans lequel au moins une surface de contact entre les éléments d'accouplement (19,20,21) est en forme de dôme afin d'autoriser un mouvement angulaire.

4. Combinaison d'une jamble artificielle et d'un moyen d'ajustement de jambe artificielle, ladite jambe artificielle (15) comportant une douille de prothèse (16) agencée autour de la jambe, une cale d'écartement inférieure (17) construite pour être reliée à un pied artificiel, et un élément de connexion (18) placé entre la douille (16) et la cale d'écartement inférieure (17) l'élément de liaison (18) comportant des éléments d'accouplement (19,20,21) mobiles l'un par rapport à l'autre, les éléments extérieurs d'accouplement (19,21) de l'élément de liaison (18) étant amarrés à la douille (16) et à la cale d'écartement inférieure (17), respectivement, caractérisé en ce que ledit moyen d'ajustement comprend une première pièce (2) équipée d'éléments de fixation (5) désignés pour être fixés à un premier élément d'accouplement (19) de l'élément de liaison (18), une seconde pièce (3) construite pour supporter en déplacement un second élément d'accouplement (21) de l'élément de liaison (18) et un certain nombre d'éléments d'ajustement (7) coopérant avec lesdites première et seconde pièces (2,3) pour permettre d'ajuster les pièces.

5. Moyen selon la revendication 4, dans lequel les éléments d'ajustement (7) comportent quatre vis de réglage disposées symétriquement, dont chacune est introduite dans un orifice (8) taraudé dans l'une des pièces (2,3).

6. Moyen selon la revendication 5, dans lequel un ressort (11) est monté autour d'au moins deux des vis de réglage (7) afin d'obtenir un écartement souple entre les pièces (2,3).

7. Moyen selon l'une des revendications 4 à 6, dans lequel le moyen comprend un élément coulissant (13) agencé dans un guide transversal de la seconde pièce (3) pour supporter le second élément d'accouplement (21) et autoriser son déplacement.

8. Moyen selon la revendication 7, dans lequel ledit guide est constitué par une douille (12) agencée autour de deux vis de réglage (7) placées face à face et par deux axes adjacents (10) qui limitent le mouvement latéral de l'élément coulissant (13).

9. Moyen selon l'une des revendications 7 ou 8, dans lequel l'élément coulissant (13) comporte une ouverture rectangulaire (14) dans lequel le second élément d'accouplement (21) devra être introduit, la longueur de l'ouverture étant telle qu'elle autorise un mouvement latéral du second élément d'accouplement (21).

**Patentansprüche**

1. Verfahren zum Einstellen und Befestigen eines künstlichen Beines (15), welches Bein aus einem Prothesensockel (16) besteht, der rund um das Bein angeordnet ist, wobei ein unterer Abstandhalter (17) für eine Verbindung an einem künstlichen Fuss bestimmt ist, und sich ein Verbindungselement (18) zwischen dem Sockel (16) und dem unteren Abstandhalter (17) befindet, dadurch gekennzeichnet, dass das Verbindungselement (18) aus Kopplungsgliedern (19,20,21) besteht, die in Verhältnis zueinander verschiebbar sind, und die äusseren Kopplungsglieder (19,21) des Verbindungselementes (18) jeweils im Sockel (16) und im unteren Abstandhalter (17) verankert sind, dass ein Einstellinstrument (1) rund um das Verbindungselement (18) angeordnet ist und im angezogenen Zustand praktisch eine Bewegung der Kopplungsglieder (19,20,21) in einer beliebigen Richtung verhindert, jedoch das Einstellinstrument, das direkt auf die Kopplungsglieder (19,20, 21) einwirkt, im losen Zustand eine relative Einstellbewegung ermöglicht und bewirkt, welche Glieder derart versetzt werden, dass das künstliche Bein (15) praktisch richtig eingestellt wird, und dass das Einstellinstrument (1) zur Befestigung des Verbindungselementes (18) angezogen wird, und die Kopplungsglieder (19,20,21) danach miteinander verbunden werden, wonach das Einstellinstrument (1) entfernt wird.

2. Verfahren nach Anspruch 1, dadurch gekennzeichnet, dass mindestens eine Berührungsfläche zwischen den Kopplungsgliedern (19,20,21) flach ist, um eine seitliche Bewegung zu ermöglichen.

3. Verfahren nach den Ansprüchen 1 bis 2, dadurch gekennzeichnet, dass mindestens eine Berührungsfläche zwischen den Kopplungsgliedern (19,20,21) eine Winkelbewegung ermöglichen.

4. Vorrichtung aus einem künstlichen Bein und einem Einstellinstrument für das künstliche Bein, das (15) aus einem Prothesensockel (16) besteht, der rund um das Bein angeordnet ist, wobei ein unterer Abstandhalter (17) für eine Verbindung zu einem künstlichen Fuss ausgelegt ist, und ein Verbindungselement (18) zwischen dem Sockel (16) und dem unteren Abstandhalter (17) angeordnet ist, und das Verbindungselement (18) aus Kopplungsgliedern (19,20,21) besteht, die bezüglich einander verschiebbar sind, wobei die äusseren Kopplungsglieder (19,21) des Verbindungselementes (18) jeweils am Sockel (16) und am unteren Abstandhalter (17) befestigt sind, dadurch gekennzeichnet, dass das Einstellinstrument einen ersten Teil (2), der mit Gliedern (5) zur Befestigung an einem ersten Kopplungsglied (19) des Verbindungselementes (18) versehen ist, einen zweiten Teil (3), der als eine verschiebbare Stütze eines zweiten Kopplungsgliedes (18) ausgelegt ist, und eine Anzahl von Einstellkörpern (7) umfasst, die mit dem ersten und dem zweiten Teil (2,3) Zusammenwirken, um eine Einstellung der Teile zu ermöglichen.

5. Vorrichtung nach Anspruch 4, dadurch gekennzeichnet, dass die Einstellkörper (7) vier symmetrisch angeordnete Schrauben umfasst, die durch ein Gewindeloch (8) in einen der Teile (2,3) gehen.

6. Vorrichtung nach Anspruch 5, dadurch gekennzeichnet, dass eine Feder (11) rund um mindestens zwei der Einstellschrauben (7) zur Bildung eines elastischen Abstandes zwischen den Teilen (2,3) vorgesehen ist.

7. Vorrichtung nach einem der Ansprüche 4 bis 6, dadurch gekennzeichnet, dass sie ein Gleitelement (13) in einer Querführung am zweiten Teil (3) zur Stütze des zweiten Kopplungsgliedes (21) sowie zu deren Verschiebung einschliesst.

8. Vorrichtung nach Anspruch 7, dadurch gekennzeichnet, dass die Führung aus einer Durchführung (12), die rund um zwei gegeneinander positionierte Einstellschrauben (7) angeordnet ist, und aus zwei benachbarten Stiften (10) besteht, die eine seitliche Bewegung des Gleitelementes (13) begrenzt.

9. Vorrichtung nach Anspruch 7 oder 8, dadurch gekennzeichnet, dass das Gleitelement (13) mit einer rechtwinkligen Öffnung (14) versehen ist, in welche das zweite Kopplungsglied (21) eingesetzt werden soll, wobei die enge der Öffnung derart bemessen ist, dass sie eine seitliche Bewegung des zweiten Kopplungsgliedes (21) ermöglicht.

FIG. 1

FIG. 2

FIG. 3